# EUROPÄISCHE PATENTSCHRIFT

(11) **EP 1 406 852 B1**
(45) Veröffentlichungstag und Bekanntmachung des Hinweises auf die Patenterteilung: **10.11.2004**
(21) Anmeldenummer: 02738032.8
(22) Anmeldetag: 04.05.2002
(51) Int. Cl.: C07B 41/04, C07B 43/04, C07C 41/06, C07C 209/60, C07C 43/15, C07C 43/215

(54) **VERFAHREN ZUR TELOMERISATION VON NICHT CYCLISCHEN OLEFINEN**
METHOD FOR TELOMERIZING NON-CYCLIC OLEFINS
PROCEDE DE TELOMERISATION D'OLEFINES NON CYCLIQUES

(30) Priorität: 09.06.2001 DE 10128144
(43) Veröffentlichungstag der Anmeldung: 14.04.2004
(73) Patentinhaber: Oxeno Olefinchemie GmbH, 45772 Marl (DE)
(72) Erfinder: RÖTTGER, Dirk, 45657 Recklinghausen (DE); BELLER, Matthias, Professor Dr., 18211 Nienhagen (DE); JACKSTELL, Ralf, Dr., 27472 Cuxhaven (DE); KLEIN, Holger, 18069 Rostock (DE); WIESE, Klaus-Diether, 45721 Haltern (DE)
(74) Vertreter: Lang, Arne, Dr.
(86) Internationale Anmeldenummer: PCT/EP2002/004909
(87) Internationale Veröffentlichungsnummer: WO 2002/100803

(56) Entgegenhaltungen:
- WO-A-91/09822
- DE-A- 2 151 662
- US-A- 4 831 183
- US-A- 5 834 611
- R. JACKSTELL: "A highly efficient catalyst for the telomerization of 1,3-dienes with alcohols: first synthesis of a monocarbenepalladium(0)-olefin complex" ANGEWANDTE CHEMIE INTERNATIONAL EDITION., Bd. 41, Nr. 6, 15. März 2002 (2002-03-15), Seiten 986-989, XP001111654 WEINHEIM DE

## Beschreibung

Die vorliegende Erfindung betrifft ein Verfahren zur Telomerisation von nicht cyclischen Olefinen mit mindestens zwei konjugierten Doppelbindungen (I) mit Nukleophilen (II) wobei als Katalysator ein Palladiumkomplex eingesetzt wird.

Unter Telomerisation wird im Rahmen dieser Erfindung die Umsetzung von Olefinen mit konjugierten Doppelbindungen (konjugierte Diene) in Gegenwart eines Nucleophils (Telogens) verstanden. Als Hauptprodukte werden dabei Verbindungen erhalten, die sich aus zwei Äquivalenten des Diens und einem Äquivalent des Nucleophils aufbauen.

Die Produkte der Telomerisationsreaktion haben technische Bedeutung als vielseitig einsetzbare Vorstufen für Lösemittel, Weichmacher, Feinchemikalien und Wirkstoffvorprodukte. Die aus Butadien erhältlichen Verbindungen Octadienol, Octadienylether oder Octadienylester sind potentielle Zwischenprodukte in Verfahren zur Darstellung von entsprechenden Alkenen.

Die Telomerisation von Dienen mit Nukleophilen ist eine technisch interessante Methode zur Veredelung von kostengünstigen, industriell verfügbaren Dienen. Von besonderem Interesse ist aufgrund der guten Verfügbarkeit die Verwendung von Butadien, Isopren oder von diese Diene enthaltenden Cracker-Schnitten. Bis dato wird die Telomerisation von Butadien jedoch lediglich von der Firma Kuraray im Feinchemikalienbereich zur Synthese von 1-Octanol praktisch angewendet. Gründe, die den breiteren Einsatz von Telomerisationsprozessen verhindern, sind unter anderem mangelnde Katalysatoraktivitäten, Katalysatorproduktivitäten und Selektivitätsprobleme von Telomerisationskatalysatoren. Somit führen die bekannten Telomerisationsprozesse zu hohen Katalysatorkosten und/oder Nebenprodukten, die eine großtechnische Realisierung verhindern.

Als wirksame Katalysatoren für die Telomerisation haben sich halogenfreie Palladium(0)- sowie Palladium(II)-Verbindungen erwiesen (A. Behr, in *"Aspects* of *Homogeneous Catalysis";* Herausgeber R. Ugo, D. Reidel Publishing Company, Doordrecht/Boston/Lancaster, **1984,** Vol. 5, 3). Daneben wurden auch Verbindungen anderer Übergangsmetalle, wie z. B. Cobalt (R. Baker, A. Onions, R. J. Popplestone, T.N. Smith, J. *Chem. Soc., Perkin Trans.* // **1975**, 1133-1138), Rhodium, Nickel (R. Baker, D.E. Halliday, T.N. Smith, J. *Organomet. Chem.* **1972,** 35, C61-C63; R. Baker, *Chem. Rev.* **1973,** 73, 487-530; R. Baker, A.H. Cook, T.N Smith, J. *Chem. Soc., Perkin Trans. ll* **1974,** 1517-1524.) und Platin, als Katalysatoren eingesetzt. Die letztgenannten Systeme sind jedoch Palladiumkomplexen hinsichtlich Aktivität und Selektivität unterlegen.

Die Telomerisation ist in der Fachliteratur ausführlich beschrieben. Die bekannten oben genannten Katalysatoren liefern beispielsweise bei der Telomerisation von Butadien mit Methanol generell Gemische der aufgeführten Produkte **1a, 1b, 2, 3** mit X = O, R¹ = Me. Hauptprodukte sind dabei die gewünschten technisch wichtigen linearen Telomere **1a** und **1b.** Jedoch entstehen signifikante Anteile des verzweigten Telomers **2** und von 1,3,7-Octatnen **3.**

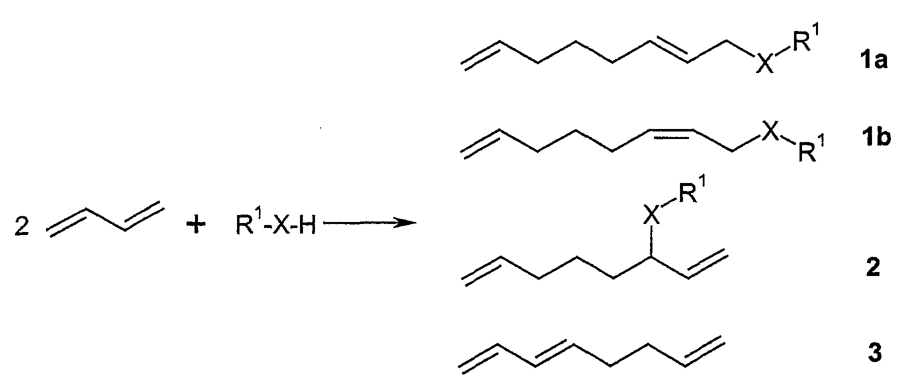

Weiterhin entstehen 4-Vinyl-1-cyclohexen (Diels-Alder-Produkt des Butadiens) in variablen Ausbeuten sowie - in der Regel in nur geringen Mengen - weitere Nebenprodukte. Dieses Spektrum von Produkten findet man generell auch bei Einsatz anderer Nucleophile mit aktiven H-Atomen, wobei an Stelle der Methoxygruppe die entsprechenden Reste des jeweiligen Nucleophils treten.

Die signifikante Bildung der genannten Nebenprodukte ist ein weiterer Grund, der eine Umsetzung eines wirtschaftlichen und umweltfreundlichen Verfahren außerordentlich schwierig macht. So konnten, obwohl die Telomerisation von Butadien mit Methanol bereits von mehreren Firmen intensiv bearbeitet und patentiert wurde, die oben genannten Probleme nicht befriedigend gelöst werden.

In einem von Dow Chemical in WO 91/09822 im Jahr 1989 beschriebenen kontinuierlichen Verfahren mit Palladiumacetylacetonat / 2 Äquivalenten Triphenylphosphan als Katalysator wurden Katalysatorproduktivitäten (turnover numbers) bis zu 44000 erzielt. Allerdings sind die Chemoselektivitäten bei derartigen Katalysatorumsatzzahlen für das Zielprodukt **1** < 85 %.

National Distillers and Chem. Corp. (US 4,642,392, US 4,831,183) beschrieben 1987 ein diskontinuierliches Verfahren zur Herstellung von Octadienylethern. Dabei wurde das Produktgemisch destillativ vom Katalysator (Palladiumacetat / 5 Äq. Triphenylphosphan) abgetrennt, der in Tetraglyme gelöst zurückbleibt. Der Katalysator kann bis zu zwölfmal wiederverwendet werden, wobei jeweils Phosphan ergänzt wird. Der Startansatz lieferte den linearen Ether allerdings in nur 57 % Ausbeute (entspricht TON 2000). Das n/iso-Verhältnis von Produkt 1 zu Produkt 2 beträgt in diesem Fall nur 3.75 : 1. In einem weiteren Patent von National Distillers wurde das Produktgemisch durch Extraktion mit Hexan von der Reaktionslösung abgetrennt. Die Telomerisation wurde dabei in Dimethylformanid oder Sulfolan mit dem Katalysatorgemisch Palladim(II)acetat / 3 Äq. Triphenylphosphinmonosulfonat durchgeführt. Der erste Ansatz lieferte das lineare Telomer mit einer TON von 900. Die Selektivität bezüglich des linearen Alkohols betrug geringe 40 %.

Auch längerkettige primäre Alkohole wie Ethanol, Propanol und Butanol (J. Beger, H. Reichel, *J. Prakt. Chem.* **1973,** *315*, 1067) bilden mit Butadien die entsprechenden Telomere. Allerdings ist die Katalysatoraktivität der bekannten Katalysatoren hier noch geringer als in den oben genannten Fällen. So wurden unter identischen Reaktionsbedingungen [Pd(acetylacetonat)₂ / PPh₃ / Butadien / Alkohol = 1 : 2 : 2000 : 5000; 60 °C / 10 h] die Telomere von Methanol mit 88 % Ausbeute, diejenigen von Propanol mit 65 % Ausbeute und von Nonanol nur noch mit 21 % Ausbeute gebildet.

Zusammenfassend kann man sagen, dass die bekannten Palladiumphosphankatalysatoren für Telomerisationsreaktionen von Butadien mit Alkoholen keine befriedigenden katalytische Wechselzahlen (Katalysatorproduktivitäten, "turnover numbers" = TON) aufweisen. Technisch angestrebte Produktivitäten von > 100.000 sind mit bekannten Systemen nicht oder kaum beschrieben. Dabei sollten gleichzeitig hohe Selektivitäten von > 95% Chemound Regioselektivität erreicht werden, um ein ökologisch vorteilhaftes Verfahren zu erzielen.

Carbonsäuen sind wie Alkohole geeignete Nucleophile in Telomerisationsreaktionen. Aus Essigsäure und Butadien erhält man in guten Ausbeueten die entsprechenden Octadienylderivate **1a, 1b** und **2** mit R = Me-CO, X = O (DE 2 137 291). Das Verhältnis der Produkte **1/2** kann über die Liganden am Palladium beeinflusst werden (D. Rose, H. Lepper, *J. Organomef. Chem.* **1973,** 49, 473). Mit Triphenylphosphin als Ligand wurde ein Verhältnis 4/1 erreicht, bei Einsatz von Tris(o-methylphenyl)phosphit konnte das Verhältnis auf 17/1 gesteigert werden. Andere Carbonsäuren wie Pivalinsäure, Benzoesäure oder Methacrylsäure, aber auch Dicarbonsäuren lassen sich ebenfalls mit Butadien umsetzen.
Shell Oil hat aufbauend auf die Telomerisation von konjugierten Dienen mit Carbonsäuren ein Verfahren zur Herstellung von α-Olefinen in der US 5 030 792 beschrieben.

Telomerisationsreaktionen, bei denen Wasser als Nucleophil eingesetzt wird, sind unter anderem von der Firma Kuraray intensiv untersucht worden (US 4 334 117, US 4 356 333, US 5 057 631). Dabei werden Phosphine, meistens wasserlösliche Phosphine, oder Phosphoniumsalze (EP 0 296 550) als Liganden eingesetzt. Der Einsatz von wasserlöslichen Diphosphinen als Ligand wird in WO 98 08 794 beschrieben, DE 195 23 335 schützt die Umsetzung von Alkadienen mit Wasser in Gegenwart von Phosphonit oder Phosphinitliganden.

Die Telomerisation von Butadien mit Nucleophilen, wie Formaldehyd, Aldehyden, Ketonen, Kohlendioxid, Schwefeldioxid, Sulfinsäuren, β-Ketoestern, β-Diketonen, Malonsäureestern, α-Formylketonen und Silanen ist ebenfalls beschrieben.

Der größere Teil der Arbeiten zur Telomerisation wurde mit Butadien durchgeführt. Die Reaktion ist aber auch auf andere Diene mit konjugierten Doppelbindungen anwendbar. Diese kann man formal als Derivate des Butadiens betrachten, in dem Wasserstoffatome durch andere Gruppen ersetzt sind. Technisch bedeutsam ist vor allem Isopren. Da Isopren im Gegensatz zum Butadien ein unsymmetrisches Molekül ist, kommt es bei der Telomerisation zur Bildung von weiteren Isomeren (J. Beger, Ch. Duschek, H. Reichel, J. *Prakt. Chem.* **1973,** *315*, 1077-89). Das Verhältnis dieser Isomeren wird dabei erheblich durch die Art des Nucleophils und auch die Wahl der Liganden beeinflusst.

Aufgrund der genannten Bedeutung Telomerisationsprodukte und den Problemen des derzeitigen Stands der Technik, besteht ein großer Bedarf nach neuen Katalysatorsystemen für Telomerisationsreaktionen, die kostengünstige stabile Liganden aufweisen, die nicht die Nachteile der bekannten katalytischen Verfahren zeigen, die für die großtechnische Durchführung geeignet sind und die Telomerisationsprodukte in hoher Ausbeute, Katalysatorproduktivität und Reinheit liefern.

Diese Aufgabe wird gelöst durch ein Verfahren zur Telomerisation von nicht cyclischen Olefinen mit mindestens zwei konjugierten Doppelbindungen (**I**) oder Mischungen, die solche Olefine enthalten, mit Nukleophilen **(II),** wobei als Kalalysator ein Palladiumcarbenkomplex verwendet wird.

In einer bevorzugten Ausführungsform werden als Nucleophile **(II)** Verbindungen der allgemeinen Formel **(IIa)** oder **(IIb)** eingesetzt,

R¹-O-H (**IIa**),

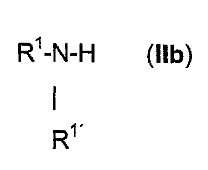

worin R¹, R^{1'} unabhängig voneinander ausgewählt sind aus Wasserstoff, einer linearen, verzweigten oder cyclischen C₁ bis C₂₂-Alkylgruppe, einer Alkenylgruppe, einer Alkinylgruppe, einer Carboxylgruppe oder einer C₆ bis C₁₈ Arylgruppe, wobei diese Gruppen Substituenten ausgewählt aus der Gruppe -CN, -COOH, -COO-Alkyl-(C₁-C₈), -CO-Alkyl-(C₁-C₈), -Aryl-(C₆-C₁₀), -COO-Aryl-(C₆-C₁₀), - CO-Aryl-(C₆-C₁₀), -O-Alkyl-(C₁-C₈), -O-CO-Alkyl-(C₁-C₈), -N-Alkyl₂-(C₁-C₈), -CHO, - SO₃H, -NH₂, -F, -Cl, -OH, -CF₃, -NO₂ enthalten können
und wobei die Reste R¹, R^{1'} über kovalente Bindungen miteinander verknüpft sein können.

Als Katalysator wird bevorzugt ein Palladiumkomplex mit Carbenliganden der Formeln (**III**) oder **(IV)** verwendet, 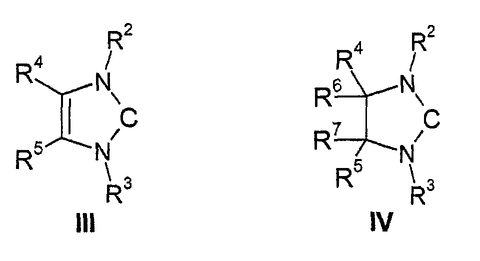 wobei R² und R³ unabhängig voneinander eine lineare, verzweigte oder cyclische C₁ bis C₂₄-Alkyl- oder C₅ bis C₁₈-Arylgruppe sind und die Alkyl- und Arylgruppe unabhängig voneinander die Substituenten -CN, -COOH, COO-Alkyl-(C₁-C₈), -CO-Alkyl-(C₁-C₈), -Aryl-(C₅-C₁₈), -Alkyl-(C₁-C₂₄), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, - F, -Cl, -OH, -CF₃, -NO₂, Ferrocenyl enthalten können,
und wobei R⁴ bis R⁷ unabhängig voneinander Wasserstoff, -CN, -COOH, -COO-Alkyl-(C₁-C₈), -CO-Alkyl-(C₁-C₈), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃, - NO₂ oder eine lineare, verzweigte oder cyclische C₁ bis C₂₄-Alkyl- oder C₆ bis C₁₈-Arylgruppe ist und die Alkyl- und Arylgruppe unabhängig voneinander die Substituenten -CN, -COOH, -COO-Alkyl-(C₁-C₈), -CO-Alkyl-(C₁-C₈), -Aryl-(C₆-C₁₀), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃, -NO₂, enthalten können, wobei die Reste R⁴ und R⁵ auch Teil eines verbrückenden aliphatischen oder aromatischen Ringes sein können.

In der Telomerisation können prinzipiell alle nicht cyclischen Olefine mit mindestens zwei konjugierten Doppelbindungen eingesetzt werden. Im Rahmen dieser Erfindung ist der Einsatz von 1,3-Butadien und Isopren (2-Methyl-1,3-butadien) bevorzugt. Dabei können sowohl die reinen Diene als auch Mischungen, die diese Diene enthalten, eingesetzt werden.

Als 1,3-Butadien enthaltende Mischungen kommen vorzugsweise Mischungen von 1,3-Butadien mit anderen C₄-Kohlenwasserstoffen und/oder C₅-Kohlenwasser-stoffe zum Einsatz. Solche Mischungen fallen beispielsweise bei Spalt(Crack)-Prozessen zur Produktion von Ethen an, in denen Raffineriegase, Naphtha, Gasöl, LPG (liquified petroleum gas), NGL (natural gas liquid) usw. umgesetzt werden. Die bei diesen Prozessen als Nebenprodukt anfallenden C₄-Schnitte enthalten je nach CrackVerfahren unterschiedliche Mengen an 1,3-Butadien. Typische 1,3-Butadienkonzentrationen im C₄-Schnitt, wie sie aus einem Naphtha-Steamcracker erhalten werden, liegen bei 20 - 70 % 1,3-Butadien.

Die C₄-Komponenten n-Butan, i-Butan, 1-Buten, cis-2-Buten, trans-2-Buten und i-Buten, die ebenfalls in diesen Schnitten enthalten sind, stören die Umsetzung im Telomerisationsschritt nicht oder nur unwesentlich.

Diese mit kumulierten Doppelbindungen (1,2-Butadien, Allen usw.) und Alkine, insbesondere Vinylacetylen, können hingegen als Moderatoren in der Telomerisationsreaktion wirken. Es ist daher vorteilhaft, die C4-Alkine und ggf. das 1,2-Butadien vorher zu entfernen (DE 195 23 335). Dies kann, falls möglich, über physikalische Verfahren wie Destillation oder Extraktion erfolgen. Auf chemischem Weg können die Alkine über Selektivhydrierungen zu Alkenen oder Alkanen und die kumulierten Diene zu Monoenen reduziert werden. Verfahren für derartige Hydrierungen sind Stand der Technik und zum Beispiel in WO 98/12160, EP-A-0 273 900, DE-A-37 44 086 oder US 4 704 492 beschrieben.

Als Nucleophile werden bevorzugt alle Verbindungen eingesetzt werden, die der allgemeinen Formel **II** genügen. Beispiele für Telogene nach der allgemeinen Formel **II** sind
- Wasser,
- Monoalkohole und Phenole wie zum Beispiel Methanol, Ethanol, n-Propanol, Isopropanol, Allylalkohol, Butanol, Octanol, 2-Ethylhexanol, Isononanol, Benzylalkohol, Cyclohexanol, Cyclopentanol, 2-Methoxyethanol, Phenol oder 2,7-Octadien-1-ol
- Dialkohole wie zum Beispiel Ethylenglycol, 1,2-Propandiol, 1,3-Propandiol, 1,4-Butandiol, 1,2-Butandiol, 2,3-Butandiol und 1,3-Butandiol
- Polyole wie zum Beispiel Glycerin, Glucose, Saccharose,
- Hydroxyverbindungen wie zum Beispiel α-Hydroxyessigsäureester
- Carbonsäuren wie zum Beispiel Essigsäure, Propansäure, Butansäure, Isobutansäure, Benzoesäure, 1,2-Benzoldicarbonsäure, 1,3-Benzoldicarbonsäure, 1,4-Benzoldicarbonsäure, 1,2,4-Benzoltricarbonsäure,
- Ammoniak,
- primäre Amine wie zum Beispiel Methylamin, Ethylamin, Propylamin, Butylamin, Octylamin, 2,7-Octadienylamin, Dodecyclamin, Anilin, Ethylendiamin oder Hexamethylendiamin
- sekundäre Amine wie Dimethylamin, Diethylamin, N-Methylanilin, Bis(2,7-Octadienyl)amin, Dicyclohexylamin, Methylcyclohexylamin, Pyrrolidin, Piperidin, Morpholin, Piperazin oder Hexamethylenimin.

Telogene, die selbst über eine Telomerisationsreaktion erhalten werden können, können direkt eingesetzt oder aber in situ gebildet werden. So kann beispielsweise 2,7-Octadien-1-ol aus Wasser und Butadien in Anwesenheit des Telomerisationskatalysators in situ gebildet werden, 2,7-Octadienylamin aus Ammoniak und 1,3-Butadien usw.

Besonders bevorzugt eingesetzte Telogene sind Wasser, Methanol, Ethanol, n-Butanol, Allylalkohol, 2-Methoxyethanol, Phenol, Ethylenglycol, 1,3-Propandiol, Glycerin, Glucose, Saccharose, Essigsäure, Butansäure, 1,2-Benzoldicarbonsäure, Ammoniak, Dimethylamin und Diethylamin.

Als Lösemittel findet im allgemeinen das eingesetzte Nucleophil Verwendung, wenn es bei Reaktionsbedingungen als Flüssigkeit vorliegt. Es können jedoch auch andere Lösemittel eingesetzt werden. Die eingesetzten Lösemittel sollten dabei weitgehend inert sein. Bevorzugt wird der Zusatz von Lösemitteln bei Einsatz von Nucleophilen, die unter Reaktionsbedingungen als Feststoffe vorliegen oder bei Produkten, die unter den Reaktionsbedingungen als Feststoffe anfallen würden. Geeignete Lösemittel sind unter anderem aliphatische, cycloaliphatische und aromatische Kohlenwasserstoffe wie zum Beispiel C₃-C₂₀-Alkane, Mischungen niederer Alkane (C₃-C₂₀), Cyclohexan, Cyclooctan, Ethylcyclohexan, Alkene und Polyene, Vinylcyclohexen, 1,3,7-Octatrien, die C₄-Kohlenwasserstoffe aus Crack-C₄-Schnitten, Benzol, Toluol und Xylol; polare Lösemittel wie zum Beispiel tertiäre und sekundäre Alkohole, Amide wie zum Beispiel Acetamid, Dimethylacetamid und Dimethylformamid, Nitrile wie zum Beispiel Acetonitril und Benzonitril, Ketone wie zum Beispiel Aceton, Methylisobutylketon und Diethylketon; Carbonssäureester wie zum Beispiel Essigsäureethylester, Ether wie beispielsweise Dipropylether, Diethylether, Dimethylether, Methyloctylether, 3-Methoxyoctan, Dioxan, Tetrahydrofuran, Anisol, Alkyl- und Arylether von Ethylenglycol, Diethylenglycol und Polyethylenglycol und andere polare Lösemittel wie zum Beispiel Sulfolan, Dimethylsulfoxid, Ethylencarbonat, Propylencarbonat und Wasser. Auch Ionische Flüssigkeiten, beispielsweise Imidazolium oder Pyridiniumsalze, können als Lösemittel eingesetzt werden.
Die Lösemittel kommen allein oder als Mischungen verschiedener Lösemittel zum Einsatz.

Die Temperatur, bei der die Telomerisationsreaktion ausgeführt wird, liegt zwischen 10 und 180 °C, bevorzugt zwischen 30 und 120 °C, besonders bevorzugt zwischen 40 und 100 °C. Der Reaktionsdruck beträgt 1 bis 300 bar, bevorzugt 1 bis 120 bar, besonders bevorzugt 1 bis 64 bar und ganz besonders bevorzugt 1 bis 20 bar.

Essentiell für das erfindungsgemäße Verfahren ist, dass die Telomerisationsreaktion mit Katalysatoren auf Basis von Palladium-Komplexen mit Carbenliganden durchgeführt wird.

Beispiele für Carbenliganden, die den allgemeinen Formeln **III** oder **IV** entsprechen, und Komplexe, die derartige Liganden enthalten sind in der Fachliteratur bereits beschrieben (W. A. Herrmann, C. Köcher, *Angew. Chem.* **1997,** *109,* 2257; Angew. Chem. Int. Ed. Engl. **1997,** 36, 2162; V.P.W. Böhm, C.W.K. Gstöttmayr, T. Weskamp, W.A. Herrmann, *J. Organomet. Chem.* **2000,** *595*, 186; DE 44 47 066).

Im Rahmen dieser Erfindung werden unter Carbenliganden sowohl freie Carbene, die als Ligand fungieren können, als auch an Palladium koordinierte Carbene verstanden.

Das Katalysatormetall Palladium, aus dem die sich unter Reaktionsbedingungen die aktiven Katalysatoren bilden, kann auf verschiedene Weisen in den Prozess eingebracht werden.
a) Als Palladium-Carbenkomplexe, wobei das Palladium bevorzugt in den Oxidationsstufen (II) oder (0) vorliegt.
b) In Form von Palladiumvorstufen, aus denen in situ die Katalysatoren gebildet werden.

### Zu a)

Beispiele sind Palladium(0)carben-olefin-Komplexe, Palladium(0)dicarbenkomplexe und Palladium(II)dicarbenkomplexe, Palladium(O)carben-1,6-dien-Komplexe. Als 1,6-Dien können beispielsweise Diallylamin, 1,1'-Divinyltetramethyldisiloxan, 2,7-Octadienylether oder 2,7-Octadienylamine fungieren. Weitere Beispiele zeigen die nachfolgenden Formeln I-a bis I-e.

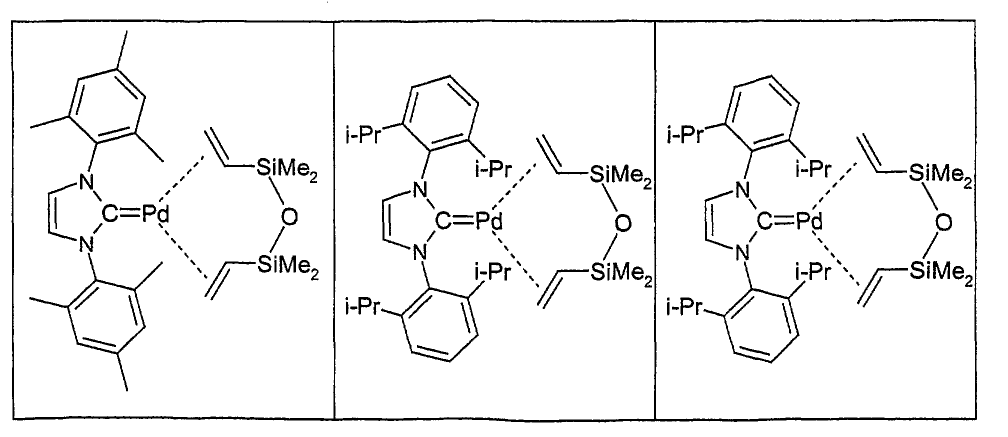

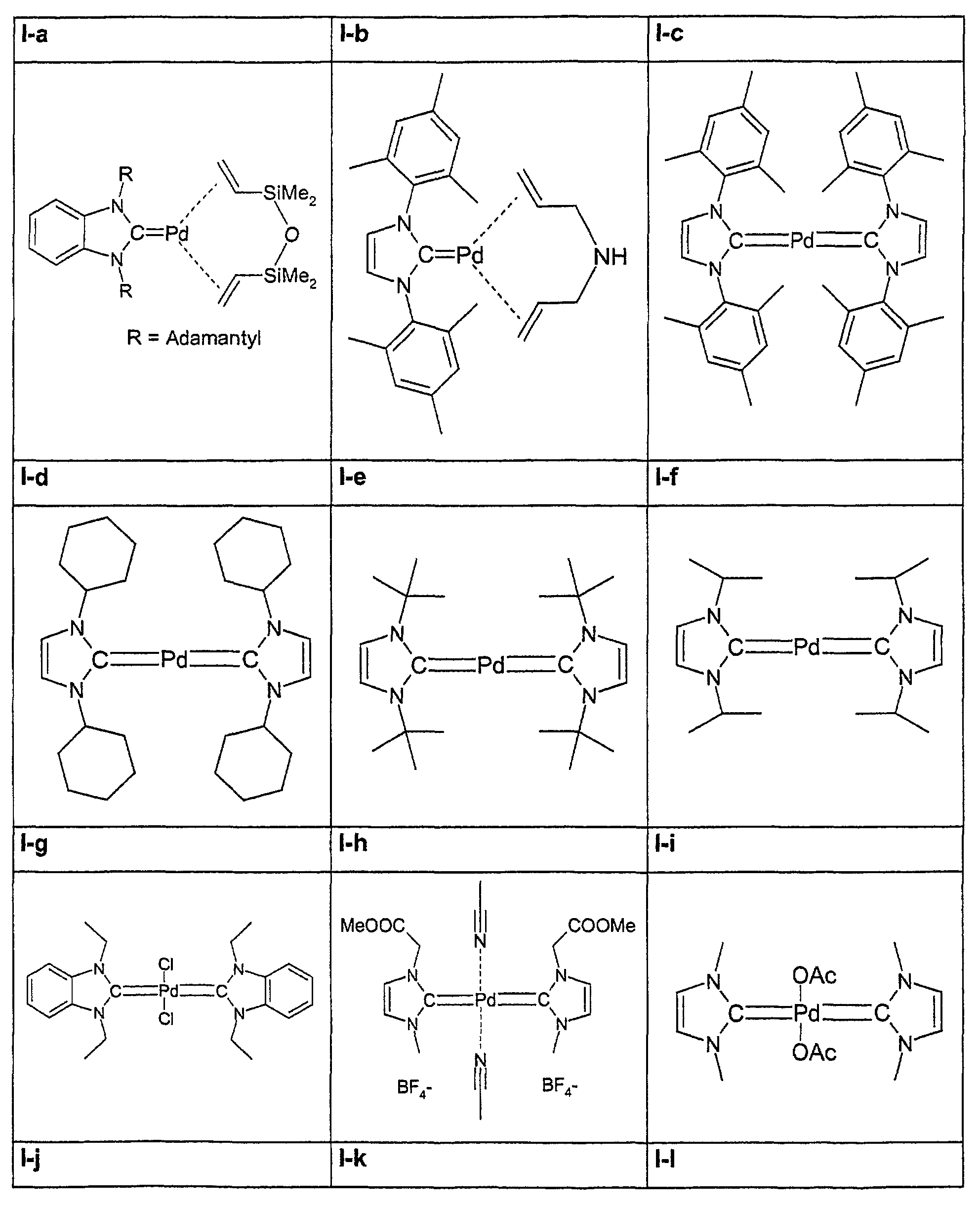

Die Carbenkomplexe des Palladiums können auf verschiedenste Weisen dargestellt werden. Ein einfacher Weg ist beispielsweise die Anlagerung von Carbenliganden oder der Austausch von Liganden an Palladiumkomplexen durch Carbenliganden. So sind beispielsweise die Komplexe **I-f** bis **I-i** durch Austausch der Phosphorliganden des Komplexes Bis(tri-o-tolylphosphin)palladium(0) erhältlich (T. Weskamp, W.A. Herrmann, J. *Organomet. Chem.* **2000,** 595, 186).

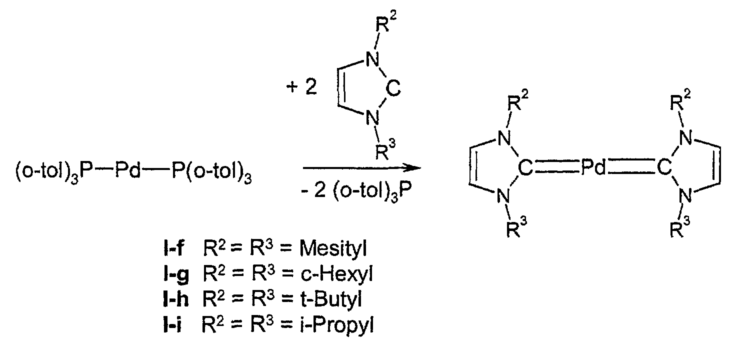

### Zu b)

Als Palladiumvorstufen können beispielsweise eingesetzt werden: Palladium(II)acetat, Palladium(II)chlorid, Palladium(II)bromid, Lithiumtetrachloropalladat, Palladium(II)acetylacetonat, Palladium(0)-dibenzylidenaceton-Komplexe, Palladium(II)propionat, Palfadium(II)chloridbisacetonitril, Palladium(II)-bistriphenylphosphandichlorid, Palladium(II)chloridbisbenzonitril, Bis(tri-o-tolylphosphin)-palladium(0) und weitere Palladium(0)- und Palladium(II)-Komplexe.

Die Carbene nach den allgemeinen Formeln **III** und **IV** werden in Form freier Carbene oder als Metallkomplexe eingesetzt oder in situ aus Carbenvorstufen erzeugt.

Als Carbenvorstufen eignen sich beispielsweise Salze der Carbene gemäß den allgemeinen Formeln **V** und **VI,** 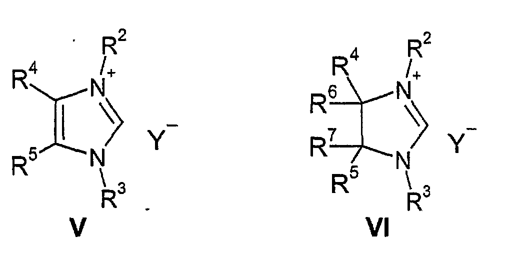 wobei R², R³, R⁴, R⁵, R⁶, R⁷ dieselbe Bedeutung haben wie in Formeln III und IV und Y für eine einfach geladene anionische Gruppe oder entsprechend der Stöchiometrie anteilig für eine mehrfach geladene anionische Gruppe steht.

Beispiele für Y sind Halogenide, Hydrogensulfat, Sulfat, Alkylsulfate, Arylsulfate, Borate, Hydrogencarbonat, Carbonat, Alkylcarboxylate, Arylcarboxylate.

Aus den Salzen der Carbene können die entsprechenden Carbene beispielsweise durch Umsetzung mit einer Base freigesetzt werden.

Die Konzentration des Katalysators, formal angegeben in ppm (Masse) an Palladium-Metall bezogen auf die Gesamtmasse, beträgt 0.01 ppm bis 1000 ppm, bevorzugt 0.5 bis 100 ppm, besonders bevorzugt 1 bis 50 ppm.
Das Verhältnis [Mol/Mol] von Carben zu Pd beträgt 0.01:1 bis 250:1, bevorzugt 1:1 bis 100:1, besonders bevorzugt 1:1 bis 50:1. Neben den Carbenliganden können noch weitere Liganden, beispielsweise Phosphorliganden wie Triphenylphosphin, in der Reaktionsmischung vorliegen.

Aufgrund der Katalysatoraktivitäten und -stabilitäten ist es bei dem erfindungsgemäßen Verfahren möglich, extrem kleine Mengen an Katalysator zu verwenden. Neben einer Verfahrensführung, bei der der Katalysator wiederverwendet wird, wird so auch die Option eröffnet, den Katalysator nicht zu recyceln. Beide Varianten sind in der Patentliteratur bereits beschrieben (WO 90/13531, US 5254782, US 4642392).

Oftmals ist es vorteilhaft, die Telomerisationsreaktion in Gegenwart von Basen durchzuführen. Bevorzugt werden basische Komponenten mit einem pK_{b}-Wert kleiner 7, insbesondere Verbindungen ausgewählt aus der Gruppe Amine, Alkalinetallsalze, Erdalkalinetallsalze eingesetzt.

Als basische Komponente sind beispielsweise geeignet Amine wie Trialkylamine, die alicyclisch oder/und offenkettig sein können, Amide, Alkali- oder/und Erdalkalisalze aliphatischer oder/und aromatischer Carbonsäuren, wie Acetate, Propionate, Benzoate bzw. entsprechende Carbonate, Hydrogencarbonate, Alkoholate von Alkaliund/oder Erdalkalielementen, Phosphate, Hydrogenphosphate oder/und Hydroxide bevorzugt von Lithium, Natrium, Kalium, Calcium, Magnesium, Cäsium, Ammoniumund Phosphoniumverbindungen. Bevorzugt sind als Zusatz Hydroxide der Alkali- und Erdalkalielemente und Metallsalze des Nucleophils nach der allgemeinen Formel **II**.

Im allgemeinen wird die basische Komponente zwischen 0.01 mol% und 10 mol% (bezogen auf das Olefin), bevorzugt zwischen 0.1 mol% und 5 mol% und ganz besonders bevorzugt zwischen 0.2 mol% und 1 mol% eingesetzt.

In dem erfindungsgemäßen Verfahren beträgt das Verhältnis [Mol/Mol] zwischen eingesetztem Dien und Nucleophil 1:100 bis 100:1, bevorzugt 1:50 bis 10:1, besonders bevorzugt 1:10 bis 2:1.

Das erfindungsgemäße Verfahren kann kontinuierlich oder diskontinuirelich betrieben werden und ist nicht auf den Einsatz bestimmter Reaktortypen begrenzt. Beispiele für Reaktoren, in denen die Reaktion durchgeführt werden kann, sind Rührkesselreaktor, Rührkesselkaskade, Strömungsrohr und Schlaufenreaktor. Auch Kombinationen verschiedener Reaktoren sind möglich, beispielsweise ein Rührkesselreaktor mit nachgeschaltetem Strömungsrohr.

Bei dem erfindungsgemäßen Verfahren werden erstmalig Carbenliganden bei Telomerisationsreaktionen eingesetzt. Überraschenderweise übertreffen die erfindungsgemäßen Katalysatoren die bekannten Palladium-Phosphan-Katalysatoren sowohl in der Selektivität als auch Produktivität. Bei dem erfindungsgemäßen Verfahren können beispielsweise bei der Telomerisation von Butadien mit Alkoholen problemlos Turnover-Werte der Katalysatoren (Katalysatorproduktivitäten) in der Größenordnung von 200.000 und mehr realisiert werden.

Die folgenden Beispiele sollen die Erfindung näher erläutern, ohne den Schutzbereich der Patentanmeldung zu beschränken.

### Beispiele

Allgemeine Arbeitsvorschrift: zur Telomerisation von Butadien mit Methanol: In einem 100-ml-Schlenkrohr werden unter Schutzgas eine entsprechende Menge Katalysator (zwischen 0.01 und 0.0001 mol%) in 56 g (1.75 mol) Methanol gelöst. Die Lösung wird mit 5 mmol Triethylamin oder Natriumhydroxid versetzt. Anschließend wird die Reaktionslösung in den evakuierten Autoklaven eingesaugt, der Autoklav auf T<-10°C gekühlt und Butadien einkondensiert (Mengenbestimmung durch Massenverlust in der Butadienvorratsflasche). Der Autoklav wird auf Reaktionstemperatur erwärmt und nach der Reaktion auf Raumtemperatur abgekühlt. Nicht umgesetztes Butadien wird in eine mit Trockeneis gekühlte Kühlfalle zurückkondensiert. Aus der Massenzunahme der Reaktionslösung wird der Umsatz bestimmt. Zur Isolierung des Produktes wird die Lösung im Vakuum destilliert.

**GC-Analytik:** Die Reaktionslösung wurde mit 5 ml Isooctan (a) oder 5 ml Diethylenglycoldimethylether (b) (GC-Standard) versetzt .

### 2,7-Octadienyl-1-methylether:

**GC** (Säule HP 5/30 m, Temp.-Programm: 35 °C, 10 min, mit 8 °C min⁻¹ auf 280 °C, Inj.: 250 °C, konst. Fluß, a). t_{R}(Vinylcyclohexen) = 12.3 min, t_{R}(Octatrien) = 11.6 min und 11.7 min, t_{R}(1) = 19 min, t_{R}(Isooctan) = 4.5 min. 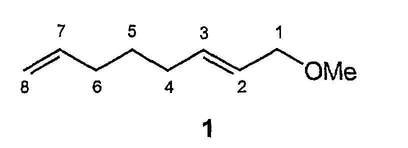 ^{**1**}**H NMR** (CDCl₃, 400 MHz)
δ = 1.39 (quint, ³J_{5,4} und ₆ = 8 Hz, 2H, 5-H), 1.9 (m, 4H, 4-H und 6-H), 3.2 (s, 3H, OCH₃), 3.7 (d, ³J_{1,2} = 6 Hz, 2H, 1-H), 4.75-4.9 (m, 2H, 8-H), 5.35 - 5.45 (m, 1 H, 7-H), 5.5 - 5.7 (m, 2H, 2-H und 3-H).
^{**13**}**C NMR** (CDCl₃, 100 MHz):
δ = 28.6 (C-5), 32.0 (C-4), 33.5 (C-6), 57.9 (OCH₃), 73.5 (C-1), 114.9 (C-8), 126.9 (C-2), 134.8 (C-3), 138.8 (C-7).

### 2,7-Octadienyl-1-butylether:

**GC** (Säule HP 5/30 m, Temp.-Programm: 35 °C, 10 min, mit 8 °C min⁻¹ auf 280 °C, Inj.: 250 °C, konst. Fluß, b). t_{R}(Vinylcyclohexen) = 12 min, t_{R}(Octatrien) = 11.6 min und 11.7 min, t_{R}(2) = 24.1 min, t_{R}(Diglyme) = 17.1 min. 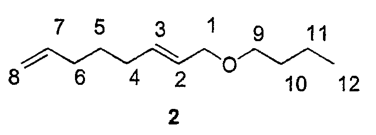 ^{**1**}**H NMR** (CDCl₃, 400 MHz)
δ=0.75 (t, J = 7.3 Hz, 3H, 12-H), 1.25 (sext, J = 7.1 Hz, 2H, 11-H), 1.39 (q, ³J_{5,4} und ₆ = 7 Hz, 2H, 5-H), 1.42 (quint, J = 7.1 Hz, 2H, 10-H), 1.9 (m, 4H, 4-H und 6-H), 3.26 (t, J = 6.7 Hz, 2H, 9-H), 3.7 (dd, J = 6 Hz, J = 1 Hz , 2H, 1-H), 4.76-4.9 (m, 2H, 8-H), 5.36 - 5.45 (m, 1 H, 7-H), 5.5 - 5.7 (m, 2H, 2-H und 3-H).
^{**13**}**C NMR** (CDCl₃, 100 MHz):
δ = 13.6 (C-12), 19.1 (C-11 ), 28.05 (C-5), 31.4 (C-10), 31.6 (C-4), 32.9 (C-6), 69.1 (C-9), 71.3 (C-1), 114.3 (C-8), 126.7 (C-2), 133.5 (C-3), 138.2 (C-7).

MS m/z (%): 182 [M⁺] (1.4), 139 (4.3), 126 (10.6), 108 (24), 101 (3.9), 97 (11), 93 (27), 82 (35), 67 (72), 57 (100); HRMS: berechnet für C₁₂H₂₂O: 182.16707, gefunden: 182.16460

### Versuchsbeispiele 1 - 17:

Die Telomerisation erfolgte analog zu der allgemeinen Arbeitsvorschrift zur Telomerisation von Butadien mit Methanol als Alkohol. Bei Einsatz anderer Alkohole wurde die Masse an Alkohol beibehalten und die Menge an Butadien, Katalysator u.s.w. entsprechend den Angaben in der Tabelle angepaßt. Als Palladiumverbindungen wurden die Komplexe A-E zugegeben. Als Base wurde Natriumhydroxid eingesetzt, die Reaktionsdauer betrug jeweils 16 Stunden.

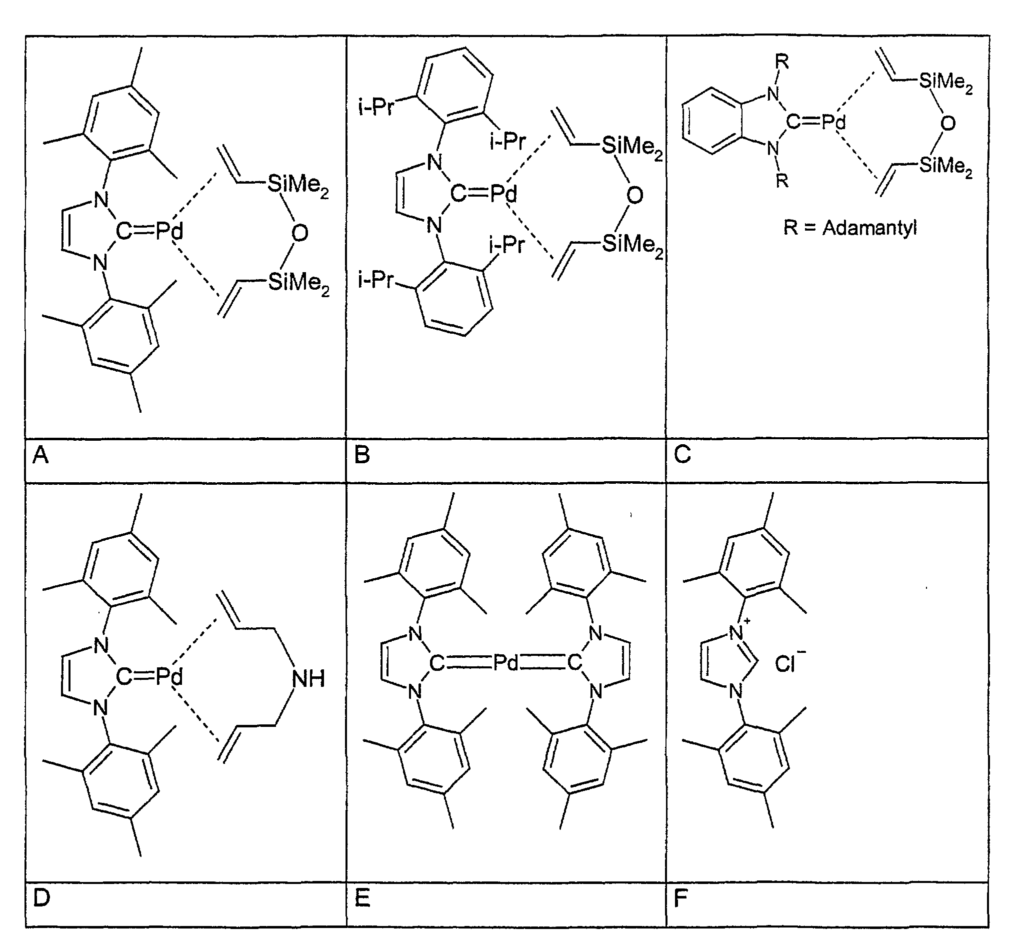

### Beispiel 18 - Synthese des Palladiumkomplexes E.

Zu einer Suspension von 1 g Bis(tri-o-tolylphosphin)palladium(0) in 20 ml Toluol gibt man eine Lösung von vom 915 mg 1,3-Dimesitylimidazolin-2-yliden gelöst in 20 ml Toluol. Die Reaktionsmischung wird bei Raumtemperatur eine Stunden gerührt und anschließend wird das Lösungsmittel im Vakuum entfernt. Nach Waschen des Rückstands mit Hexan (3 x 10 ml) und Trocknung im Vakuum erhält man den Komplex **E**, der ohne weitere Reinigung in den Telomerisationsreaktionen eingesetzt wurde.
Ausbeute 65%, ^{**13**}**C NMR** (C₆D₃, 100 MHz): δ = 186.2 (Pd-CN2).

### Beispiele 19 - 20

Der Katalysator wird in situ aus einer Palladiumvorstufe und der Carbenvorstufe **F** generiert [Pd(OAc)₂ - Palladium(II)acetat, Pd₂(dba)₃ = Di(dibenzylidenaceton)-palladium(0)]. Verbindung **F** kann über bekannte Vorschriften erhalten werden (beispielsweise WO 0001739) und ist auch kommerziell erhältlich (Strem).
Die Telomerisation erfolgte nach der allgemeinen Arbeitsvorschrift zur Telomerisation von Butadien mit Methanol. Als Base wurde Natriumhydroxid eingesetzt, die Reaktionsdauer betrug jeweils 16 Stunden.

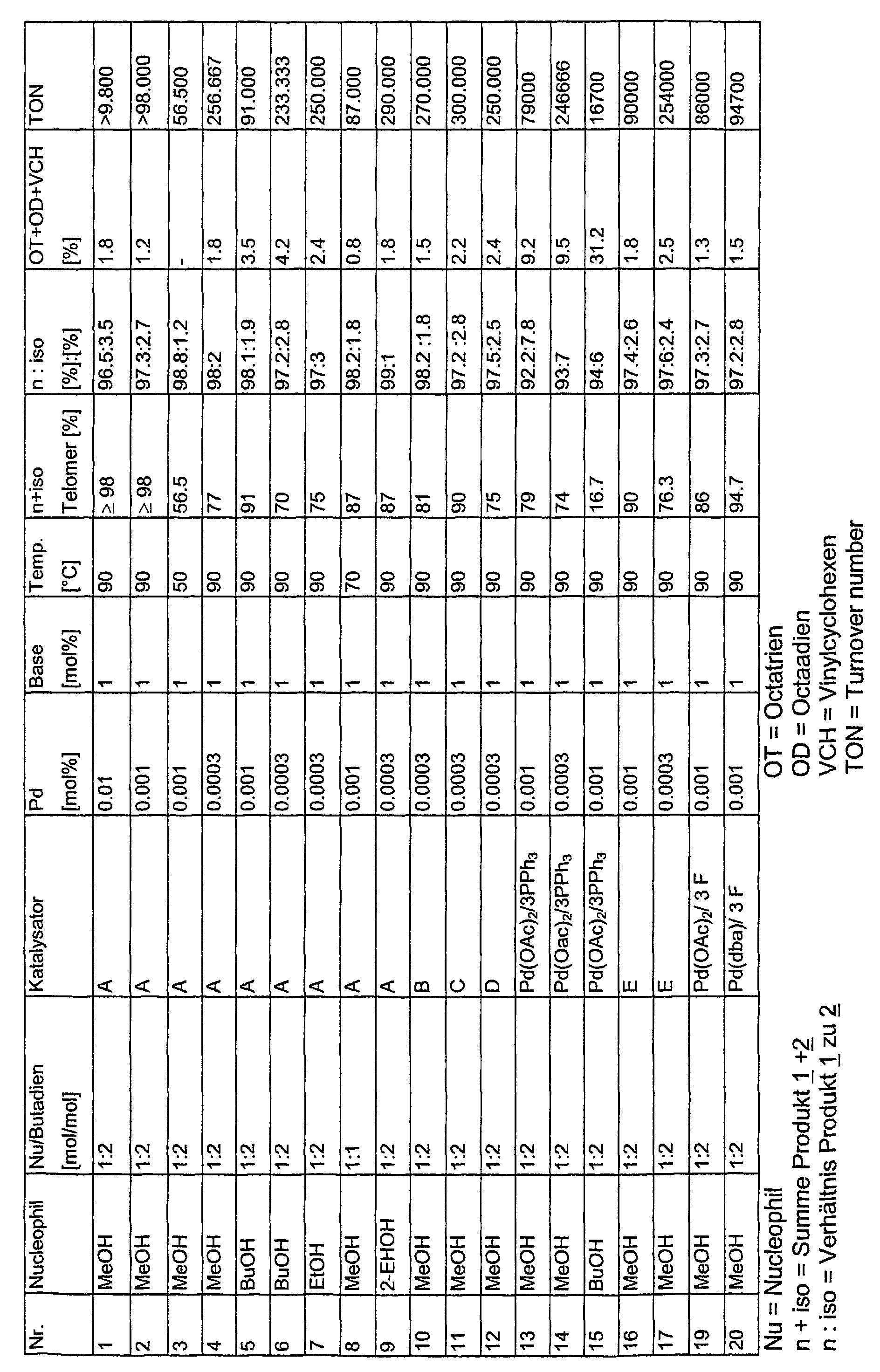

### Beispiele 21-22

In einem 100-ml-Schlenkrohr werden unter Schutzgas 70.6 g (0,75 mol) Phenol und die entsprechende Menge des Katalysators **A** (mol% Pd bezogen auf mol Butadien) in 70 ml Tetrahydrofuran gelöst. Als Base wird Natriumphenolat hinzugegeben, 1 mol% bezogen auf die eingesetzte Menge an Phenol. Anschließend wird die Reaktionslösung in den evakuierten Autoklaven eingesaugt, der Autoklav auf T<-10°C gekühlt und Butadien einkondensiert (Mengenbestimmung durch Massenverlust in der Butadienvorratsflasche). Das molare Verhältnis Phenol zu Butadien betrug 2 zu 1.

Der Autoklav wird auf 90°C erwärmt und nach 16 Stunden auf Raumtemperatur abgekühlt. Nicht umgesetztes Butadien wird in eine mit Trockeneis gekühlte Kühlfalle zurückkondensiert. Aus der Massenzunahme der Reaktionslösung wird der Umsatz bestimmt. Zur Isolierung des Produktes wird die Lösung im Vakuum destilliert.

| **Nr.** | **Base** | **Pd [mol%]** | **n+iso Telomer [%]** | **n:iso [%]:[%]** | **OT+VCH [%]** | **TON** |
|---|---|---|---|---|---|---|
| **21** | NaOPh | 0.005 | 56 | 89:11 | 1.3 | 11200 |
| **22** | NaOPh | 0.001 | 6.4 | 95:5 | 3.4 | 6400 |

### 2,7-Octadienyl-1-phenylether:

^{**1**}**H NMR** (CDCl₃, 400 MHz)
δ =1.8 (quin, J = 7.5, 5-H), 2.3-2.4 (m, 4H, 4-H und 6-H), 4.74 (d,d, J = 5.5, J = 1, 2H, 1-H), 5.2-5.35 (m, 2H, 8-H), 5.9 - 6.2 (m, 3H, 7-H, 2-H, 3-H), 7.18-7.21 (m, 3H, 10-H, 12-H), 7.5-7.6 (m, 2H, 11-H)
^{**13**}**C NMR** (CDCl₃, 100 MHz):
δ = 28.0 (C-5), 31.6 (C-4), 33.0 (C-6), 68.4 (C-1), 114.53 (C-8), 120.5 (C-11), 125.1 (C-2), 129.1 (C-12), 129.2 (C-10), 134.8 (C-3), 138.3 (C-7), 158.5 (C-9)
MS m/z (%): [M⁺] 202 (2.5), 108 (9.9), 94 (100), 79 (11), 67 (55), 58 (11), 55 (24), 43 (40), HRMS: berechnet für C₁₄H₁₈O: 202.13577, gefunden: 202.13485

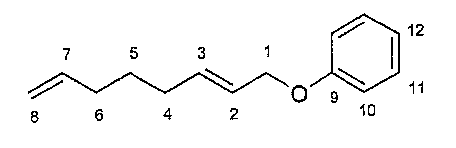

### Beispiel 23

Die Telomerisation erfolgte analog zu der allgemeinen Arbeitsvorschrift zur Telomerisation von Butadien mit Methanol. Als Palladiumverbindung wurden der Komplex **A** zugegeben. Als Base wurde Natriumisopropylat, 1 mol%, eingesetzt. Die Reaktionsdauer betrug 16 Stunden bei 90°C, das molare Verhältnis i-Propanol zu Butadien betrug 2 zu 1.

| **Nr.** | **Kataly- sator** | **Pd [mol%]** | **n+iso Telomer [%]** | **n :iso [%]:[%]** | **OT+VCH [%]** | **TON** |
|---|---|---|---|---|---|---|
| **23** | A | 0.005 | 72.5 | 82:18 | 26.5 | 14500 |

### 2,7-Octadienyl-1-isopropylether:

**GC** (Säule HP 5/30 m, Temp.-Programm: 35°C, 10 min, mit 8 °C min⁻¹ auf 280 °C, Inj.: 250 °C, konst. Fluß, b). t_{R}(Vinylcyclohexen) = 12 min, t_{R}(Octatrien) = 11.6 min und 11.7 min, t_{R}(**2**) = 19.2 min, t_{R} (1 )= 16.51, t_{R}(Diglyme) = 17.1 min.
^{**1**}**H NMR** (CDCl₃, 400 MHz)
δ = 1.05 (d of s, 6H, 10-H, 11-H), 1.4 (quint, J = 7.5 Hz, 2H, 10-H), 1.9 (m, 4H, 4-H und 6-H), 3.5 (sept, J = 6.1 Hz, 2H, 9-H), 3.82 (dd, *J* = 6.2 Hz, *J* = 1 Hz , 2H, 1-H), 4.76-4.9 (m, 2H, 8-H), 5.36 - 5.45 (m, 1 H, 7-H), 5.5 - 5.75 (m, 2H, 2-H und 3-H).
^{**13**}**C NMR** (CDCl₃, 100 MHz):
δ = 21.7 (C-11, C-10 ), 27.9 (C-5), 31.3 (C-4), 32.9 (C-6), 69.1 (C-9), 70.8 (C-1), 114.8 (C-8), 127.7 (C-2), 133.5 (C-3), 138.7 (C-7).
MS *m*/*z* (%): (M⁺] 168(0.11), 126 (12.5), 109 (30.6), 97 (13), 93 (25), 82 (68), 67 (95), 55 (76), 43 (100)
EA: berechnet für C₁₁H₂₀O: C: 78.51, H: 11.98, , gefunden: C: 78.56, H: 11.95

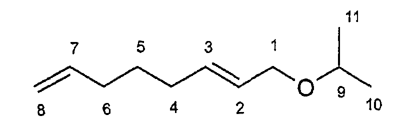

### Beispiel 24 - Synthese von 1,3-Bis(2,4,6-trimethyl-phenyl)imidazolium-tosylat (G)

1.5 g (4.4 mmol) 1,3-Bis(2,4,6-trimethyl-phenyl)imidazoliumchlorid **(F)** werden in 10 ml absolutem MeOH gelöst mit 0.854 g (4.4 mmol) Natriumtosylat versetzt. Nach vollständigem Auflösen (Magnetrührung) des Natriumtosylates wird im Vakuum bis auf ca 3 ml Lösungsvolumen eingeengt und es werden 50 ml Aceton zugesetzt. Es wird zwei Stunden bei 40 °C gerührt, das ausgefallene Natriumchlorid abfiltriert und die Lösung auf ca. 10 ml im Vakuum eingeengt. Nach 24 h werden die ausgefallenen weißen Kristalle abfiltriert und mit 5 ml Aceton gewaschen und im Vakuum getrocknet. Die Ausbeute beträgt 1.9 g (90 %). M = 476.63 g/Mol. Über diese Methode der Umsalzung kann das Chlorid-Anion auch gegen diverse andere Anionen, beispielsweise Carboxylat-Anionen, ausgetauscht werden.
¹H-NMR (δ[ppm], J[Hz], MeOH-d₄): 2.17 s (12H), 2.29 s (3H), 2.34 s (6H), 3.92 s (2H), 7.1 d (2H, J = 8.5), 7.13 s (4H), 7.6 d (2H, J = 8.5), 8.1 s (2H)
¹³C-NMR (δ[ppm], MeOH-d₄): 143.1 s, 140.4 s, 138.1 s, 133.6 s, 130.1 s, 128.6 s, 127.3 s, 124.9 s ,124.1 s

### Beispiele 25-28

Die Telomerisation erfolgte analog zu der allgemeinen Arbeitsvorschrift zur Telomerisation von Butadien mit Methanol. Es wurden jeweils 15.0 g 1,3-Butadien, 17.8 g Methanol, 0.00127 g Tris(dibenzylidenaceton)dipalladium(0) und 0.11 g Natriumhydroxid eingesetzt. Als Liganden wurden eingesetzt 1,3-Bis(2,4,6-trimethylphenyl)imidazolium-chlorid (**F**) und 1,3-Bis(2,4,6-trimethyl-phenyl)imidazolium-tosylat (**G**). Reaktionen wurden bei 50°C und 90°C durchgeführt, die Reaktionsdauer betrug jeweils 16 Stunden.

| **Nr.** | **Ligand** | **Ligand/Pd** | **Temp.** | **Ausbeute** | **n :iso** | **Ausbeute** |
|---|---|---|---|---|---|---|
| | | **[mol/mol]** | **[°C]** | **n+iso Telomer [%]** | **[%]:[%]** | **OT+VCH [%]** |
| 25 | **F** | 4/1 | 50 | 29.6 | 98.5:1.5 | 0.3 |
| 26 | **G** | 4/1 | 50 | 35.0 | 98.5:1.5 | 0.3 |
| 27 | **F** | 2/1 | 90 | 94.3 | 97.5:2.5 | 1.4 |
| 28 | **G** | 2/1 | 90 | 92.0 | 97.6:2.4 | 1.0 |

## Patentansprüche

1. Verfahren zur Telomerisation von nicht cyclischen Olefinen mit mindestens zwei konjugierten Doppelbindungen (**I**) oder Mischungen, die solche Olefine enthalten, mit Nukleophilen (**II**), wobei als Katalysator ein Palladiumcarbenkomplex verwendet wird.

2. Verfahren nach Anspruch 1, **dadurch gekennzeichnet, dass** als Nukleophile solche ausgewählt aus der Gruppe Wasser, Alkohole, Phenole, Polyole, Carbonsäuren, Ammoniak, primäre oder sekundäre Amine eingesetzt werden.

3. Verfahren nach den Ansprüchen 1 oder 2, **dadurch gekennzeichnet, dass** als Nucleophile **(II)** Verbindungen der allgemeinen Formel **(IIa)** oder **(IIb)** eingesetzt werden,
R¹-O-H (**IIa**), 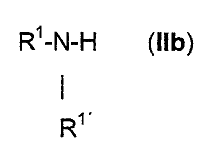
worin R¹, R^{1'} unabhängig voneinander ausgewählt sind aus Wasserstoff, einer linearen, verzweigten oder cyclischen C₁ bis C₂₂-Alkylgruppe, einer Alkenylgruppe, einer Alkinylgruppe, einer Carboxylgruppe oder einer C₅ bis C₁₈ Arylgruppe, wobei diese Gruppen Substituenten ausgewählt aus der Gruppe -CN, -COO_{H}, -COO-Alkyl-(C₁-C₈), -CO-Alkyl-(C₁-C₈), -Aryl-(C₅-C₁₀), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃, - NO₂ enthalten können, und wobei die Reste R¹, R^{1'} über kovalente Bindungen miteinander verknüpft sein können.

4. Verfahren nach den Ansprüchen 1 oder 2, **dadurch gekennzeichnet, dass** ein Palladiumcarbenkomplex mit Carbenliganden der Formeln **(III)** oder **(IV)** verwendet wird, 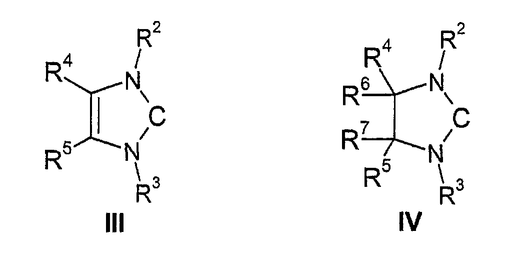 wobei R² und R³ unabhängig voneinander eine lineare, verzweigte oder cyclische C₁ bis C₂₄-Alkylgruppe oder eine C₅ bis C₁₈-Arylgruppe sind, wobei die Alkylgruppe und die Arylgruppe unabhängig voneinander die Substituenten - CN, -COOH, -COO-Alkyl-(C₁-C₈), -CO-Alkyl-(C₁-C₈), -Aryl-(C₆-C₁₈), -Alkyl-(C₁-C₂₄), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃, -NO₂, Ferrocenyl enthalten können,
und wobei R⁴ bis R⁷ unabhängig voneinander Wasserstoff, -CN, -COOH, -COO-Alkyl-(C₁-C₈), -CO-Alkyl-(C₁-C₈), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, - NH₂, -F, -Cl, -OH, -CF₃, -NO₂ oder eine lineare, verzweigte oder cyclische C₁ bis C₂₄-Alkylgruppe oder C₆ bis C₁₈-Arylgruppe ist und die Alkylgruppe und Arylgruppe unabhängig voneinander die Substituenten -CN, -COOH, -COO-Alkyl-(C₁-C₈), -CO-Alkyl-(C₁-C₈), -Aryl-(C₆-C₁₀), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃, -NO₂, enthalten kann, und wobei die Reste R⁴ und R⁵ auch Teil eines verbrückenden aliphatischen oder aromatischen Ringes sein können.

5. Verfahren nach den Ansprüchen 1 bis 4, **dadurch gekennzeichnet, dass** als nicht cyclisches Olefin 1,3-Butadien oder Isopren eingesetzt wird.

6. Verfahren nach den Ansprüchen 1 bis 5, **dadurch gekennzeichnet, dass** 1,3-Butadien in Mischung mit anderen C4-Kohlenwasserstoffen oder C5-Kohlenwasserstoffen eingesetzt wird.

7. Verfahren nach den Ansprüchen 1 bis 6, **dadurch gekennzeichnet, dass** als Lösemittel das Nucleophil (**II**) oder inerte organische Lösemittel oder Mischungen derselben eingesetzt werden.

8. Verfahren nach den Ansprüchen 1 bis 7, **dadurch gekennzeichnet, dass** die Reaktion bei Temperaturen von 10 bis 180 °C und einem Druck von 1 bis 300 bar durchgeführt wird.

9. Verfahren nach den Ansprüchen 1 bis 8, **dadurch gekennzeichnet, dass** das Verhältnis von Carbenligand zu Pd [Mol/Mol] 0,01:1 bis 250:1 beträgt.

10. Verfahren nach den Ansprüchen1 bis 9, **dadurch gekennzeichnet, dass** die Palladiumcarbenkomplexe als isolierte Komplexe eingesetzt werden.

11. Verfahren nach den Ansprüchen 1 bis 10, **dadurch gekennzeichnet, dass** die Palladiumcarbenkomplexe während der Telomerisationsreaktion in situ erzeugt werden.

12. Verfahren nach den Ansprüchen 1 bis 11, **dadurch gekennzeichnet, dass** der Reaktion katalytische Mengen einer basischen Komponente mit einem pK_{b}-Wert < 7 zugesetzt werden.

13. Verfahren nach den Ansprüchen 1 bis 12, **dadurch gekennzeichnet, dass** als basische Komponente Verbindungen ausgewählt aus der Gruppe Amine, Alkalimetallsalze, Erdalkalimetallsalze oder Mischungen derselben eingesetzt werden.

14. Verfahren nach den Ansprüchen 1 bis 13, **dadurch gekennzeichnet, dass** die basische Komponente in Mengen von 0.01 mol% bis 10 mol% bezogen auf die Olefinverbindung eingesetzt wird.

15. Verfahren nach den Ansprüchen 1 bis 14, **dadurch gekennzeichnet, dass** die Palladiumkonzentration in der Reaktionsmischung 0.01 bis 1000 ppm beträgt.

## Claims

1. A process for the telomerization of an acyclic olefin having at least two conjugated double bonds (I) or a mixture comprising such olefins by means of a nucleophile (II) using a palladium-carbene complex as catalyst.

2. A process according to claim 1, **characterized in that** the nucleophile used is selected from the group consisting of water, alcohols, phenols, polyols, carboxylic acids, ammonia and primary and secondary amines.

3. A process according to either of claims 1 and 2, **characterized in that** the nucleophile (II) used is a compound of the formula (IIa)or(IIb),
R¹ -O-H (IIa) 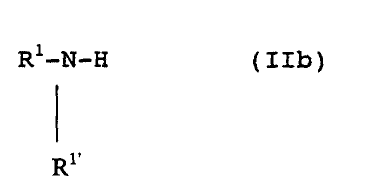
where R¹ , R^{1'} are selected independently from among hydrogen, linear, branched or cyclic C₁-C₂₂-alkyl groups, alkenyl groups, alkynyl groups, the carboxyl group and C₅-C₁₈-aryl groups, where these groups may bear substituents selected from the group consisting of -CN, -COOH, -COO-alkyl-(C₁-C₈), - CO-alkyl-(C₁-C₈), -aryl-(C₅-C₁₀), -COO-aryl-(C₆-C₁₀), -CO-aryl- ( C₆-C₁₀), -O-alkyl-(C₁-C₈), -O-CO-alkyl-(C₁-C₈), -N-alkyl₂-(C₁-C₈), -CHO, -SO₃H, -NH₂, - F, -Cl, -OH, -CF₃, -NO₂, and the radicals R¹, R^{1'} may be joined to one another via covalent bonds.

4. A process according to either of claims 1 and 2, **characterized in that** the palladium-carbene complex used comprises a carbene ligand of the formula (III) or (IV), 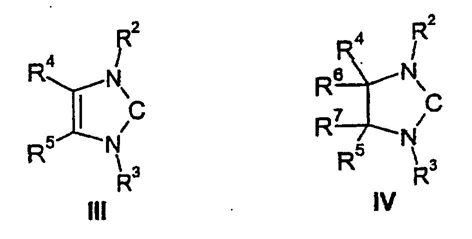 where R² and R³ are each, independently of one another, a linear, branched or cyclic C₁-C₂₄-alkyl or C₅-C₁₈-aryl group and the alkyl and aryl groups may, independently of one another, bear the substituents -CN, -COOH, COO-alkyl-(C₁-C₈), -CO-alkyl-(C₁-C₈), -aryl-(C₆-C₁₈), -alkyl-(C₁-C₂₄), -COO-aryl-(C₆-C₁₀), -CO-aryl(C₆-C₁₀),-O-alkyl-(C₁-C₈), -O-CO-alkyl-(C₁-C₈), -N-alkyl₂-(C₁-C₈), -CHO, -SO₃H, -NH₂, -F, -Cl, -OH, CF₃, -NO₂, ferrocenyl,
and R⁴ to R⁷ are each, independently of one another, hydrogen, -CN, -COOH, -COO-alkyl-(C₁-C₈), -CO-alkyl-(C₁-C₈), -CO-aryl-(C₆-C₁₀), -COO-aryl-(C₆₋ C₁₀), -O-alkyl-(C₁-C₈), -O-CO-alkyl-(C₁-C₈), -N-alkyl₂-(C₁-C₈) , -CHO, -SO₃H, -NH₂, -F, -Cl, -OH, CF₃, -NO₂ or a linear, branched or cyclic C₁-C₂₄-alkyl or C₆-C₁₈-aryl group and the alkyl and aryl groups may, independently of one another, bear the substituents -CN, -COOH, -COO-alkyl-(C₁-C₈), -CO-alkyl-(C₁- C₈), -aryl(C₆-C₁₀), -COO-aryl-(C₆-C₁₀),-CO-aryl(C₆-C₁₀), -O-alkyl-(C₁-C₈), -O-CO-alkyl-(C₁-C₈), -N-alkyl₂-(C₁-C₈), -CHO, -SO₃H, -NH₂, -F, -Cl, - OH, CF₃, -NO₂, where the radicals R⁴ and R⁵ may also be part of a bridging aliphatic or aromatic ring.

5. A process according to any of claims 1 to 4, **characterized in that** the acyclic olefin used is 1, 3-butadiene or isoprene.

6. A process according to any of claims 1 to 5, **characterized in that** 1, 3-butadiene is used in admixture with other C₄-hydrocarbons or C₅-hydrocarbons.

7. A process according to any of claims 1 to 6, **characterized in that** the nucleophile (II) or an inert organic solvent or a mixture thereof is used as solvent.

8. A process according to any of claims 1 to 7, **characterized in that** the reaction is carried out at temperatures of from 10 to 180°C and a pressure of from 1 to 300 bar.

9. A process according to any of claims 1 to 8, **characterized in that** the ratio of carbene ligand to Pd [mol/mol] is from 0.01:1 to 250:1.

10. A process according to any of claims 1 to 9, **characterized in that** the palladium-carbene complex is used as an isolated complex.

11. A process according to any of claims 1 to 10, **characterized in that** the palladium-carbene complex is generated in situ during the telomerization reaction.

12. A process according to any of claims 1 to 11, **characterized in that** a catalytic amount of a basic component having a pK_{b} of < 7 is added to the reaction.

13. A process according to any of claims 1 to 12, **characterized in that** the basic component used is a compound selected from the group consisting of amines, alkali metal salts and alkaline earth metal salts or a mixture thereof.

14. A process according to any of claims 1 to 13, **characterized in that** the basic component is used in an amount of from 0.01 mol% to 10 mol%, based on the olefin compound.

15. A process according to any of claims 1 to 14, **characterized in that** the palladium concentration in the reaction mixture is from 0.01 to 1 000ppm.

## Revendications

1. Procédé de télomérisation d'oléfines non cycliques ayant au moins deux liaisons doubles conjuguées (I) ou de mélanges qui contiennent de telles oléfines, avec des nucléophiles (II), le catalyseur utilisé étant un complexe de carbène de palladium.

2. Procédé selon la revendication 1,
**caractérisé en ce que**
comme nucléophiles, on utilise ceux choisis dans le groupe eau, alcools, phénols, polyols, acides carboxyliques, ammoniac, amines primaires ou secondaires.

3. Procédé selon les revendications 1 ou 2
**caractérisé en ce qu'**
on utilise, comme nucléophiles (II) des composés de formule générale (IIa) ou (IIb),
R¹-O-H (**IIa**), 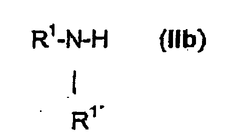
dans laquelle R¹, R^{1'}, sont choisis indépendamment l'un de l'autre parmi l'hydrogène, un groupe alkyle en C₁ à C₂₂ linéaire, ramifié ou cyclique, un groupe alcényle, un groupe alcynyle, un groupe carboxyle ou un groupe aryle en C₅ à C₁₈, ces groupes pouvant contenir des substituants choisis dans le groupe -CN, -COOH, -COO-alkyle-(C₁-C₈), - CO-alkyle-(C₁-C₈), aryle-(C₅-C₁₀), -COO-aryle-(C₆-C₁₀), -CO-aryle-(C₆-C₁₀), - O-alkyle-(C₁-C₈), -O-CO-alkyle-(C₁-C₈), -N-alkyle₂-(C₁-C₈), -CHO, -SO₃H, -NH2, -F, -CI, -OH, -CF₃, -NO₂, et dans laquelle les radicaux R¹ et R^{1'} peuvent être associés ensemble par des liaisons covalentes.

4. Procédé selon les revendications 1 ou 2,
**caractérisé en ce qu'**
on utilise un complexe de carbène de palladium ayant des ligands carbène des formules (III) ou (IV), 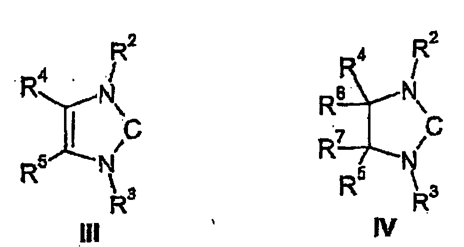 dans laquelle R² et R³, indépendamment l'un de l'autre, représentent un groupe alkyle en C₁ à C₂₄ linéaire, ramifié ou cyclique ou un groupe aryle en C₅ à C₁₈, le groupe alkyle et le groupe aryle, indépendamment l'un de l'autre, pouvant contenir les substituants -CN, -COOH, -COO-alkyle-(C₁-C₈), -CO-alkyle-(C₁-C₈), -aryle-(C₆-C₁₈), -alkyle-(C₁-C₂₄), -COO-aryle-(C₆-C₁₀), -CO-aryle-(C₆-C₁₀), -O-alkyle-(C₁-C₈), -O-CO-alkyle-(C₁-C₈), -N-alkyle₂-(C₁-C₈), -CHO, -SO₃H, -NH₂, -F, -Cl, -OH, -CF₃, - NO₂, ferrocényle,
et dans laquelle R⁴ à R⁷, indépendamment l'un de l'autre, représentent un atome d'hydrogène, un groupe -CN, -COOH, -COO-alkyle-(C₁-C₈), -CO-alkyle-(C₁-C₈), -COO-aryle-(C₆-C₁₀), -CO-aryle-(C₆-C₁₀), -O-alkyle-(C₁-C₈), -O-CO-alkyle-(C₁-C₈), -N-alkyle₂-(C₁-C₈), -CHO, -SO₃H -NH₂, -F, -Cl, -OH, -CF₃, -NO2 ou bien un groupe alkyle en C₁ à C₂₄ ou un groupe aryle en C₆ à C₁₈ linéaire, ramifié ou cyclique et le groupe alkyle et le groupe aryle, indépendamment l'un de l'autre, pouvant contenir les substituants -CN, -COOH, -COO-alkyle-(C₁-C₈), -CO-alkyle-(C₁-C₈), aryle-(C₆-C₁₀), -COO-aryle-(C₆-C-₁₀), -CO-aryle-(C₆-C₁₀), -O-alkyle-(C₁-C₈), -O-CO-alkyle-(C₁-C₈), -N-alkyle₂-(C₁-C₈), -CHO, -SO₃H, -NH₂, -F, -Cl, -OH, -CF₃, -NO₂, et dans laquelle les radicaux R⁴ et R⁵ peuvent aussi représenter une partie d'un cycle aliphatique ou aromatique pontant.

5. Procédé selon les revendications 1 à 4,
**caractérisé en ce qu'**
on utilise comme oléfine non cyclique du 1,3-butadiène ou de l'isoprène.

6. Procédé selon les revendications 1 à 5,
**caractérisé en ce qu'**
on utilise le 1,3-butadiène en mélange avec d'autres hydrocarbures en C₄ ou hydrocarbures en C₅.

7. Procédé selon les revendications 1 à 6,
**caractérisé en ce qu'**
on utilise comme solvant le nucléophile (II) ou des solvants organiques inertes ou des mélanges de ceux-ci.

8. Procédé selon les revendications 1 à 7,
**caractérisé en qu'**
on réalise la réaction à des températures de 10 à 180°C et à une pression de 1 à 300 bars.

9. Procédé selon les revendications 1 à 8,
**caractérisé en que**
le rapport de ligand carbène au Pd [mole/mole] est de 0,01:1 à 250:1.

10. Procédé selon les revendications 1 à 9,
**caractérisé en ce qu'**
on utilise les complexes de carbène de palladium en tant que complexes isolés.

11. Procédé selon les revendications 1 à 10,
**caractérisé en ce que**
les complexes de carbène de palladium sont produits in situ pendant la réaction de télomérisation.

12. Procédé selon les revendications 1 à 11,
**caractérisé en ce qu'**
on ajoute à la réaction des quantités catalytiques d'un composant basique ayant une valeur pK_{b} inférieure à 7.

13. Procédé selon les revendications 1 à 12,
**caractérisé en qu'**
on utilise, comme composant basique, des composés choisis dans le groupe amines, sels métalliques alcalins, sels métalliques alcalino-terreux ou des mélanges de ceux-ci.

14. Procédé selon les revendications 1 à 13,
**caractérisé en ce qu'**
on utilise le composant basique en quantités de 0,01 % molaire à 10 % molaire par rapport au composé d'oléfine.

15. Procédé selon les revendications 1 à 14,
**caractérisé en ce que**
la concentration de palladium dans le mélange réactionnel est de 0,01 à 1000 ppm.
